# EUROPÄISCHE PATENTSCHRIFT

(11) **EP 1 269 243 B1**
(45) Veröffentlichungstag und Bekanntmachung des Hinweises auf die Patenterteilung: **04.07.2007**
(21) Anmeldenummer: 01919424.0
(22) Anmeldetag: 04.04.2001
(51) Int. Cl.: G02B 21/00, G01N 21/53, C12M 1/34

(54) **IN-SITU-MIKROSKOPVORRICHTUNG FÜR REAKTOREN**
IN-SITU MICROSCOPE DEVICE FOR REACTORS
MICROSCOPE IN SITU POUR REACTEURS

(30) Priorität: 05.04.2000 DE 10016838
(43) Veröffentlichungstag der Anmeldung: 02.01.2003
(73) Patentinhaber: SARTORIUS AG, 37075 Göttingen (DE)
(72) Erfinder: FRERICHS, Jan-Gerd, 30459 Hannover (DE); BEHNSEN, Wilhelm, 31634 Steimbke (DE); SCHEPER, Thomas, 30559 Hannover (DE); JOERIS, Klaus, 41061 Mönchengladbach (DE); SCHAPER, Jörg, 30449 Hannover (DE)
(74) Vertreter: UEXKÜLL & STOLBERG
(86) Internationale Anmeldenummer: PCT/EP2001/003817
(87) Internationale Veröffentlichungsnummer: WO 2001/077282

(56) Entgegenhaltungen:
- EP-A- 0 497 347
- WO-A-99/36760
- DE-A- 4 032 002
- US-A- 4 364 629
- BITTNER C.; WEHNERT G.; SCHEPER T.: 'In Situ Microscopy for On-Line Determination of Biomass' BIOTECHNOLOGY AND BIOENGINEERING Bd. 60, Nr. 1, 05 Oktober 1998, Seiten 24 - 35

## Beschreibung

Die vorliegende Erfindung betrifft eine In-situ-Mikroskopvorrichtung für Reaktoren, wie etwa Bioreaktoren, mit den Merkmalen des Oberbegriffs von Patentanspruch 1.

Mit solchen In-situ-Mikroskopvorrichtungen können im laufenden Betrieb Untersuchungen an Proben des Materials im Inneren des Reaktors vorgenommen werden, beispielsweise die Konzentration bestimmter Zellen in dem Medium überwacht werden. Die Grundlagen der In-situ-Mikroskopie, für Reaktoren sind in der Patentschrift DE 40 32 002 C2 dargestellt.

Eine In-situ-Mikroskopvorrichtung mit den Merkmalen des Oberbegriffs des Anspruchs 1 ist z.B. in der Dissertation "In-situ-Mikroskopie: Ein neues Verfahren zur Online-Bestimmung der Biomasse bei Kultivierungsprozessen", Dr. Christoph Bittner, Hannover, 1994, beschrieben.

Die Überwachung und Steuerung biotechnologischer Prozesse hat in der jüngeren Vergangenheit z.B. in der chemischen und der mazeutischen Industrie eine große Bedeutung erlangt. Beispiele hierfür sind die Synthese von humanen Proteinen, wie z.B. Interleukin (IL-2), Tissue-Plasminogen-Aktivator (t-PA) oder Antithrombin (AT-III), deren Herstellung mit Hilfe der organischen Synthese nur schwer realisierbar ist, so dass die Herstellung dieser Proteine mit Hilfe der Kultivierung von Säugetierzellen bevorzugt wird. Auch bei der Herstellung von Produkten der Lebensmittelindustrie, z.B. Bier, Wein, Käse oder Brot, werden Mikroorganismen, hier insbesondere Hefen, eingesetzt. Weitere Produkte bzw. Pharmaka werden durch die Kultivierung anderer Organismen erzeugt. Zur Überwachung und Steuerung derartiger Prozesse wird bei der In-situ-Mikroskopie eine Mikroskopsonde in einen Anschlussport eines Fermenters (Reaktors) eingesetzt. Diese Mikroskopsonde ermöglicht eine Bildaufnahme direkt in der Kulturbrühe. Das aufgenommene Mikroskopbild wird mit einer an das In-situ-Mikroskop angeschlossenen CCD-Kamera aufgenommen und digitalisiert. Die Auswertung der digitalisierten mikroskopischen Bilder wird mit Hilfe von Bildverarbeitungsprogrammen auf einem Standardcomputer durchgeführt. Anhand des mit der In-situ-Mikroskopie gewonnenen Bilddatenmaterials und darauf angewendeter Analysen können Aussagen über Zellgrößen und Biomasse, Zellgrößenverteilung, Zellkonzentration, Zellmorphologie und Zellvitalität gewonnen werden. Auf Grundlage der dadurch im laufenden Betrieb gewonnenen Informationen über den Zustand des in dem Reaktor befindlichen Systems kann steuernd auf Verfahrensparameter eingewirkt werden, um eine gewünschte Entwicklung des Systems zu erzielen.

In der oben erwähnten Dissertation von Bittner wird ein In-situ-Mikroskop für die Beobachtung von Kultivierungsprozessen an Hefen beschrieben. Das Mikroskop hat ein Tauchrohr, das in einen Reaktoranschlussport eingesetzt ist. Im im Reaktorinneren liegenden Endbereich des Tauchrohrs ist ein Einlass vorgesehen, durch den das Kulturmedium frei fließen kann. Ein Mikroskopau-ßenrohr ist koaxial in dem Tauchrohr angeordnet und ist mit seinem am inneren Ende liegenden Objektiv auf eine Probenzone ßenrohr ist koaxial in dem Tauchrohr angeordnet und ist mit seinem am inneren Ende liegenden Objektiv auf eine Probenzone gerichtet, die zwischen dem Deckglas des Objektivs und einem gegenüberliegenden Objektträgerglaskörper definiert wird. Am gegenüberliegenden Ende ist das Mikroskopaußenrohr mit einer Kamera zur Aufnahme des Bildes der Probenzone verbunden. Bei offener Probenzone liegen das Objektivdeckglas und der gegenüberliegende Objektträgerglaskörper in einigem Abstand zueinander, wobei das Kulturmedium aus dem Reaktorinneren frei durch diesen Raum fließen kann. Zur Bildaufnahme wird die Probenzone geschlossen, indem der Objektträgerglaskörper auf das Objektivdeckglas zu bewegt wird, bis ein den Objektträgerglaskörper umgebender Dichtungsring in Anlage an das Objektivdeckglas kommt und so eine Probenzone mit einem definierten Volumen zwischen dem Objektträgerglaskörper und dem Objektivdeckglas schafft. Bei der bekannten Vorrichtung wird die Probenzone geschlossen, indem der Objektträgerglaskörper mit einer darunterliegenden Beleuchtungseinheit an das Objektiv herangezogen wird. Diese Bewegung wird über eine Zugstange bewirkt, die neben dem Mikroskopaußenrohr in Längsrichtung in dem Tauchrohr verläuft und an einem Ende mit der Einheit des Objektträgerglaskörpers und am anderen Ende mit einem Antrieb außerhalb des Tauchrohrs verbunden ist. Ferner ist eine Wischeinrichtung vorgesehen, mit der das Objektivdeckglas bei Bedarf durch Abwischen gereinigt werden soll. Eine solche Wischeinrichtung ist notwendig, da das Glas schnell verschmutzt und eine anderweitige Reinigung im laufenden Kultivierungsbetrieb überhaupt nicht und auch nach Abbruch des Kultivierungsprozesses nur unter erheblichem Aufwand unter komplettem Ausbau des Mikroskops aus dem Reaktor vorgenommen werden kann. Der Wischer wird ebenfalls von einem externen Antrieb über eine mechanische Kraftübertragung angetrieben.

Diese Mikroskopvorrichtung ist auch in dem Artikel "ln Situ Microscopy for On-Line Determination of Biomass" Biotechnology and Bioengeneering, Bd. 60, Nr. 1, S. 24.

Die bekannte In-situ-Mikroskopvorrichtung ist in verschiedener Hinsicht nachteilig. Zum Beispiel ist es nachteilig, dass mechanische Kraftübertragungen neben dem Mikroskoptubus durch das Tauchrohr geführt werden müssen, da dies den für den Mikroskoptubus zur Verfügung stehenden Raum einschränkt. Ferner sind solche mechanischen Kraftübertragungen konstruktiv aufwendig und störanfällig.

Der Hauptnachteil der bekannten In-situ-Mikroskopvorrichtungen besteht jedoch darin, dass im laufenden Kultivierungsbetrieb das Mikroskop nicht zugänglich ist, da beim Öffnen des Reaktors durch Ausbau des Tauchrohrs oder durch Herausziehen des Mikroskops die Kultivierung kontaminiert würde. Außerdem müsste, bei einem seitlichen Einbau des Mikroskops, der Reaktor erst leergepumpt werden, was für den praktischen Einsatz nicht in Frage kommt.

Die Entfernbarkeit des Mikroskops ist nicht nur zu Zwecken der Reinigung während des Betriebs von Bedeutung, sondern zur Sterilisation/Autoklavierung des Reaktorsystems vor der Inbetriebnahme, da dabei Temperaturen von über 120°C angewendet werden.

Für industrielle Anwendungen der In-situ-Mikroskopie werden Mikroskopvorrichtungen benötigt, die robust, flexibel und einfach zu handhaben sind.

Unter der Marke "inTrac" der Firma Mettler-Toledo GmbH sind sogenannte Wechselsonden bekannt. Dieses Anschlusssystem ist auf standardisierte Reaktoranschlüsse aufschraubbar und hat neben dem aufzuschraubenden Rohr ein darin beweglich gelagertes Tauchrohr, dessen innenliegendes Ende in das Reaktorinnere hineinschiebbar und wieder zurückziehbar ist. Das Tauchrohr ist soweit zurückziehbar, dass sein innenliegendes Ende in einer in dem Reaktoranschlussport gebildeten Spülkammer liegt, die mit Reinigungsmitteln versorgbar ist, wobei am Tauchrohr Dichtungsmittel vorgesehen sind, um bei zur Spülkammer zurückgezogenem Tauchrohr den Innenraum des Reaktors gegen die Spülkammer abgedichtet zu halten.

Es ist daher Aufgabe der vorliegenden Erfindung, eine in-situ-Mikroskopvorrichtung so auszubilden, dass eine Reinigung der Probenzone ohne Unterbrechung des Kultivierungsprozesses oder Kontamination des Reaktorinneren möglich ist.

Zur Lösung dieser Aufgabe dienen die Merkmale des Patentanspruchs 1. Vorteilhafte Ausführungsformen der Erfindung sind in den Unteransprüchen aufgeführt.

Es ist vorgesehen, dass das Tauchrohr in axialer Richtung beweglich in dem Reaktoranschlussport geführt ist, an den sich außen eine Spülkammer mit verschließbaren Öffnungen anschließt, durch die Reinigungsmittel einspeisbar sind. Das Tauchrohr kann so weit in den Anschlussport zurückgezogen werden, dass der Einlass des Tauchrohrs mit der Spülkammer kommuniziert. An dem Tauchrohr sind Dichtungsmittel vorgesehen, um bei in die Spülkammer zurückgezogenem Tauchrohr den Innenraum des Reaktors gegen die Spülkammer abgedichtet zu halten. Auf diese Weise kann die Probenzone gereinigt werden, wenn das Tauchrohr in die Spülkammer zurückgezogen ist, indem Reinigungsmittel in die Spülkammer eingespeist werden. Dabei bleibt der Reaktorinnenraum gegen das Innere der Spülkammer abgedichtet, so dass die Reinigung der Probenzone auch während eines laufenden Kultivierungsprozesses vorgenommen werden kann, ohne dass die Gefahr von Kontaminationen besteht.

Ferner kann bei in die Spülkammer zurückgezogenem Tauchrohr das Mikroskopaußenrohr herausgezogen werden. Dadurch können alle Teile der Mikroskopvorrichtung, die innerhalb des Tauchrohrs angeordnet sind, entnommen und gegebenenfalls ausgetauscht oder repariert werden, ohne dass die Sterilbarriere zum Reaktorinneren durchbrochen wird. Dadurch können auch bei laufendem Betrieb des Reaktors Änderungen an der Gestaltung oder Ausrüstung des Mikroskops, Wartungsarbeiten und dergleichen durchgeführt werden, ohne dass der Kultivierungsprozess im Reaktor unterbrochen werden müsste oder die Gefahr von Kontaminationen besteht. Dadurch lässt sich die Handhabung beim laufenden Betrieb, die Betriebssicherheit und die Variabilität durch Austausch von Komponenten des Mikroskops in entscheidendem Maße verbessern, so dass die In-situ-Mikroskopvorrichtung durch ihre Flexibilität und Robustheit für industrielle Anwendungen besonders gut geeignet ist.

In dem zum Reaktorinneren weisenden Ende des Tauchrohrs eine Beleuchtungseinheit untergebracht, die den Objektträgerglaskörper trägt und eine Lichtquelle aüfweist, um die Probenzone durch den Objektträgerglaskörper hindurch zu durchleuchten.

Gemäß einer Alternative ist das Mikroskopaußenrohr wiederum in einem Mikroskopgehäuserohr aufgenommen, wobei das Mikroskopgehäuserohr am dem Reaktorinneren zugewandten Ende bis auf einen Einlass für Probenmaterial geschlossen ist und die Probenzone rückwärtig umschließt und wobei die Beleuchtungseinheit am innen liegenden Ende des Mikroskopgehäuserohrs angeordnet ist. Dabei ist das Mikroskopaußenrohr in axialer Richtung beweglich in dem Mikroskopgehäuserohr gelagert und ist ein auf das außen liegende Ende des Mikroskopaußenrohrs einwirkende Antrieb vorgesehen. Durch den Antrieb lässt sich das Mikroskopaußenrohr relativ zum Mikroskopgehäuserohr verschieben, um so die Probenzone zwischen dem Objektivdeckglas am Mikroskopaußenrohr und dem Objektträgerglaskörper der Beleuchtungseinheit durch Vorschieben und Zurückziehen des Mikroskopaußenrohrs gesteuert durch den Antrieb schließen und öffnen zu können. Besonders vorteilhaft wird als Antrieb ein Schrittmotor oder ein geregelter Gleichstrommotor verwendet, wodurch sich eine sehr präzise Einstellung der Probenzone realisieren lässt.

Gemäß der anderen Alternative bilden die Beleuchtungseinheit und das Mikroskopaußenrohr zwei separate Einheiten, die nicht wie oben in einem gemeinsamen Mikroskopgehäuserohr aufgenommen sind, wobei die Beleuchtungseinheit am inneren Ende des skopaußenrohrs schließen und öffnen zu können.

Bei allen Ausführungsformen kann vorgesehen sein, dass ein Mikroskoptubus-verschiebbar in dem Mikroskopaußenrohr gelagert ist und dass ein Antriebsmittel vorgesehen ist, um den Mikroskoptubus in Längsrichtung gesteuert verschieben zu können, um so das Objektiv an dem Mikroskoptubus im Bezug auf die Probenzone zur Fokussierung einstellen zu können.

Mit den zuvor beschriebenen Gestaltungen ist es möglich, den im Tauchrohr zur Verfügung stehenden Querschnitt so weit wie möglich auszunutzen, da keine Antriebsübertragungen durch das Tauchrohr hindurchgeführt werden müssen. Ferner kann die Stellung von Objektträgerglaskörper und Objektivdeckglas zueinander, die zwischen sich die Probenzone bilden, sehr genau durch den Antrieb gesteuert eingestellt werden.

Dadurch, dass die Querschnittsfläche des Tauchrohrs vollständig ausgenutzt werden kann, da keine Antriebsübertragungen hindurchgeführt werden müssen, können auch Tauchrohre mit relativ kleinem Innendurchmesser Anwendung finden, in die ein Mikroskopau-ßenrohr eingeführt ist. Auf diese Weise können auch Tauchrohre von herkömmlichen, standardisierten Wechselsondensystemen eingesetzt werden, bei dem der Außendurchmesser der einzusetzenden Sonde begrenzt ist.

Wenn das Tauchrohr in die Spülkammer zurückgezogen ist, kann durch die verschließbaren Öffnungen der Spülkammer Reinigungsmittel eingespeist werden, z.B. Heißdampf, so dass auf diese Weise das Reinigungsmittel in die Probenzone durch den Einlass des Tauchrohrs eintritt, um diese zu reinigen. Wenn das Tauchrohr so weit zurückgezogen ist, dass sein Einlass mit der Spülkammer kommuniziert, ist das Reaktorinnere abgedichtet und der Einlass des Tauchrohrs liegt im Inneren der Spülkammer und hat keine Kommunikation mit dem Reaktorinneren mehr. In diesem Zustand kann das Mikroskopaußenrohr oder das Mikroskopgehäuserohr

Wenn das Tauchrohr in die Spülkammer zurückgezogen ist, kann durch die verschließbaren Öffnungen der Spülkammer Reinigungsmittel eingespeist werden, z.B. Heißdampf, so dass auf diese Weise das Reinigungsmittel in die Probenzone durch den Einlass des Tauchrohrs eintritt, um diese zu reinigen. Wenn das Tauchrohr so weit zurückgezogen ist, dass sein Einlass mit der Spülkammer kommuniziert, ist das Reaktorinnere abgedichtet und der Einlass des Tauchrohrs liegt im Inneren der Spülkammer und hat keine Kommunikation mit dem Reaktorinneren mehr. In diesem Zustand kann das Mikroskopaußenrohr oder das Mikroskopgehäuserohr aus dem Tauchrohr herausgezogen werden. Dadurch sind alle wesentlichen Teile des Mikroskops zugänglich und können repariert oder durch Austausch von Komponenten variiert werden. Die letztgenannte Möglichkeit, durch Austausch von Komponenten die Eigenschaften des Mikroskops zu verändern, betrifft auch die Probenzone, denn es kann z.B. der Objektträgerglaskörper ausgewechselt werden, um eine andere geometrische Definition der Probenzone zu erhalten.

Da ein Antrieb vorgesehen ist, um die Probenzone durch Verschieben des Mikroskopaußenrohrs oder der Beleuchtungseinheit relativ zueinander einzustellen, so ist durch einen solchen präzise steuerbaren Antrieb eine genaue Definition der Probenzone möglich. Die Motorsteuerung erlaubt es, die Probenzone variabel einzustellen, also die Höhe der Probenzone durch variables Zufahren auszuwählen. Eine ergänzende oder alternative Option, die Probenzone variabel zu gestalten, besteht in besonderen Ausgestaltungen des Objektträgerglaskörpers, die im folgenden erläutert werden.

In diesem Zusammenhang ist es insbesondere bevorzugt, dass der Objektträgerglaskörper eine Saphirglasscheibe mit ebener Außenfläche aufweist, auf der am Umfang ein ringförmiger Rand mit vorgegebener Dicke gebildet ist, der als Abstandshalter dient, sein, z.B. zwei längliche Abstandshalter, die zwischen sich eine kanalförmige, zu beiden Seiten offene Vertiefung bilden. Bei Ausführungsformen, bei denen mehrere diskrete Abstandshalter auf Abstand zueinander auf dem Objektträgerglaskörper vorgesehen sind, ist die Probenzone seitlich offen gegenüber dem umgebenden Medium, so dass dauernd Zellen durch die Probenzone strömen können und sich somit das vom Mikroskop erfasste Bild dauern ändert. Durch die Aufnahme von Bildsequenzen lässt sich so eine höhere Messfrequenz realisieren, da die Probenzone nicht für jedes Bild geöffnet und geschlossen werden muss, sondern nur in bestimmten Intervallen ein kompletter Austausch des Mediums in der Probenzone durch deren Öffnung bewirkt werden sollte. Diese Ausführungsform der Probenzone unterscheidet sich signifikant von den allseitig geschlossenen Probenzonen, die ausschließlich dazu dienen, die Probe in der Probenzone ruhig zu stellen. In der hier vorgeschlagenen Variante ist das Gegenteil der Fall. Bei Verwendung lichtstarker Leuchtdioden zur Beleuchtung kann die Belichtungszeit der Kamera so verkürzt werden, dass die Zellen, trotz ihrer Bewegung, scharf abgebildet werden.

Die erfindungsgemäße In-situ-Mikroskopvorrichtung kann mit endlicher oder unendlicher Optik arbeiten.

Ferner kann die In-situ-Mikroskopvorrichtung neben der schon erläuterten Betriebsweise der Durchlicht-Hellfeldmikroskopie mit einer Beleuchtungseinheit unterhalb der Probenzone auch zur Auflicht-Dunkelfeldmikroskopie oder zur Auflicht-Hellfeldmikroskopie verwendet werden.

Die Erfindung wird im Folgenden anhand eines Ausführungsbeispiels in den Zeichnungen erläutert, in denen:
- Figur 1: eine Teilschnittansicht einer In-situ-Mikroskopvorrichtung in Betriebsstellung im Reaktor (oben) und in zurückgezogener Stellung (unten) des Tauchrohrs im Reaktoranschlussport zeigt, wobei in der unteren Darstellung das Mikroskopgehäuserohr aus dem Tauchrohr entfernt ist;
- Figur 2: eine Teilschnittansicht der Ausführungsform aus Figur 1 zeigt, wobei das Mikroskopaußenrohr in dem Mikroskopgehäuserohr angeordnet dargestellt ist;
- Figur 3: eine Schnittdarstellung einer alternativen Ausführungsform der In-situ-Mikroskopvorrichtung einschließlich einer vergrößerten Schnittdarstellung einer Beleuchtungseinheit zeigt;
- Figur 4: verschiedene Ausführungsformen eines Objektträgerglaskörpers darstellt.

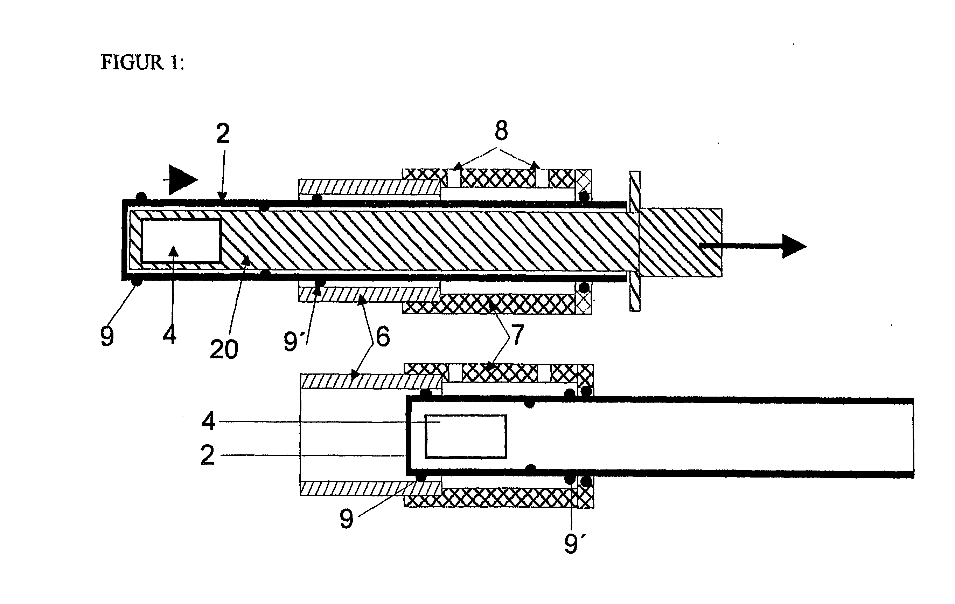

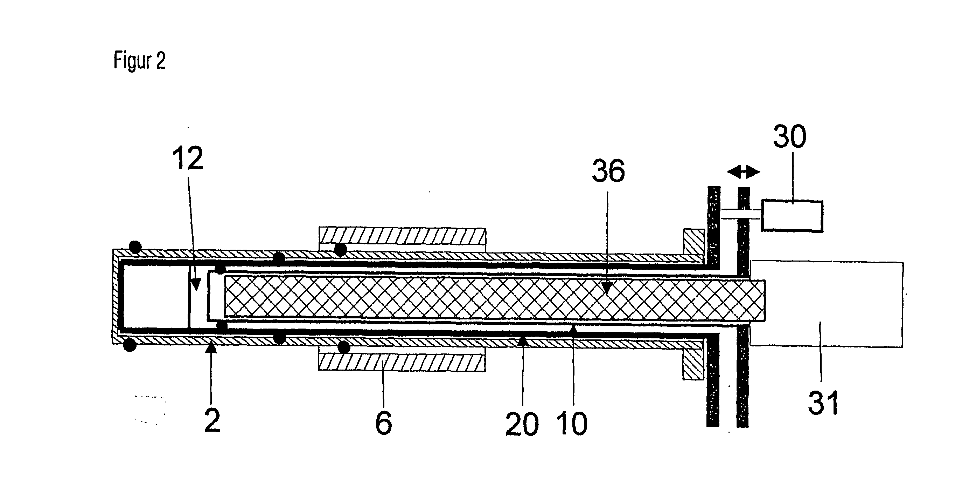

Die in den Figuren 1 und 2 im Schnitt schematisch dargestellte Ausführungsform der In-situ-Mikroskopvorrichtung hat ein Tauchrohr 2, das in einen Reaktoranschlussport 6 eingesetzt ist, der fest mit der Reaktorwand (nicht gezeigt) verbunden ist. In Figur 2 ist zur Vereinfachung der Darstellung die Spülkammer 7 am Reaktoranschlussport 6 nicht dargestellt. In der in Figur 1 oben dargestellten Stellung liegt das Tauchrohr 2 mit seinem Einlass 4 im Inneren des Reaktors, so dass das Medium im Reaktorinneren durch den Einlass 4 durch die Probenzone der In-situ-Mikroskopvorrichtung hindurchfließen kann. An dem Reaktoranschlussport 6 ist außen eine Spülkammer 7 angeschlossen, die an ihrem äußeren Ende mit Dichtungen an der Außenfläche des Tauchrohrs 2 anliegt. Das Tauchrohr 2 kann durch den Reaktoranschlussport 6 so weit zurückgezogen werden, dass der Einlass 4 mit der Spülkammer 7 kommuniziert, wie in Figur 1 unten dargestellt. In dieser Stellung dichten außen am Umfang umlaufende Dichtungen 9, die an der Innenwand des Reaktoranschlussports anliegen, das Reaktorinnere ab, so dass keine Verbindung mehr besteht zwischen dem Einlass 4 des Tauchrohrs und dem Reaktorinneren. Durch die verschließbaren Öffnungen 8 der Spülkammer 7 kann Reinigungsmittel, z.B. Heißdampf, eingespeist werden, um die Probenzone zu reinigen.

In dem Tauchrohr ist das Mikroskopgehäuserohr 20 verschiebbar gelagert. In der in Figur 1 unten dargestellten Stellung ist das Mikroskopgehäuserohr aus dem Tauchrohr entnommen, so dass alle darin befindlichen Teile des Mikroskops zur Wartung oder zum Austausch von Komponenten gehandhabt werden können. Gleichzeitig ist das Reaktorinnere durch das Tauchrohr 2 in dem Anschlussport 6 abgedichtet, so dass das Reaktorinnere nicht kontaminiert werden kann und der Kultivierungsprozess weiterlaufen kann.

Wie in Figur 2 dargestellt, befindet sich in dem Mikroskopgehäuserohr 20 ein Mikroskopaußenrohr 10, das darin verschiebbar gelagert ist. Das Mikroskopgehäuserohr 20 weist ebenfalls einen Einlass auf, der mit dem Einlass 4 des Tauchrohrs kommuniziert, so dass Medium im Reaktorinneren durch die Probenzone 12 hindurchfließen kann.

Außerhalb des Reaktors ist ein Antrieb 30 vorgesehen, z.B. ein Schrittmotor, der einen Antrieb zwischen Mikroskopgehäuserohr 20 und Mikroskopaußenrohr 10 bereitstellt, um Letzteres relativ zu dem Mikroskopgehäuserohr zu verschieben. Durch die Verschiebbarkeit des Mikroskopaußenrohrs 10 wird in dieser Ausführungsform die Bewegung realisiert, die zum Öffnen und Schließen der Probenzone verwendet wird, wobei die Probenzone zwischen einem Objektträgerglaskörper, der im Betrieb im unteren Bereich des Tauchrohrs feststehend angeordnet ist, und dem Objektivdeckglas, das am Ende des beweglichen Mikroskopaußenrohrs 10 angeordnet ist, definiert ist. Durch die verschiebbare Lagerung des Mikroskopaußenrohrs 10 und das Vorsehen eines Antriebs 30, um das Mikroskopaußenrohr 10 zum Öffnen und Schließen der Probenzone zu bewegen, kann die Querschnittsfläche im Mikroskopgehäuserohr 20 voll für das Mikroskopaußenrohr 10 ausgenutzt werden, da keinerlei mechanische Antriebsübertragungen in den vorderen Bereich der Probenzone benötigt werden.

Der Antrieb 30 setzt einerseits an einem Stempel des Mikroskopgehäuserohrs 20 und andererseits an einem Stempel des Mikroskopaußenrohrs 10 an, um diese relativ zueinander zu bewegen. Durch die Stellung des Mikroskopaußenrohrs 10 relativ zu dem Mikroskopgehäuserohr 20 wird die Größe der Probenzone, d.h. der Abstand zwischen dem Objektträgerglaskörper an der Beleuchtungseinheit und dem Objektivdeckglas an dem Mikroskopaußenrohr 10, definiert.

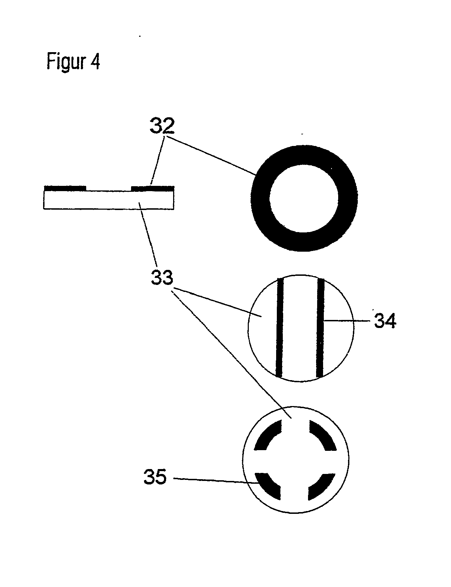

Als Antrieb 30 wird vorzugsweise ein Schrittmotor verwendet, was die Möglichkeit eröffnet, die Probenzone mit variabler Höhe jeweils den Anforderungen in einer bestimmten Situation genau anpassen zu können. Prinzipiell ist für den Antrieb 30 aber jegliches Antriebsmittel möglich, so können z.B. auch pneumatische Antriebsmittel oder ein Piezomodul für den Antrieb 30 verwendet werden. Die Motorsteuerung erlaubt es, die Probenzone variabel einzustellen, also die Höhe der Probenzone durch variables Zufahren auszuwählen. Eine weitere Möglichkeit, variable Probenzonen zu realisieren, besteht darin, Objekträgerglaskörper mit ausgewählten Abstandshaltern, die den Abstand zum Objektivdeckglas definieren, vorzusehen, wie sie weiter unten im Zusammenhang mit Figur 4 beschrieben werden.

Am äußeren Ende ist das Mikroskopaußenrohr 10 mit einer Kamera 31, z.B. einer CCD-Kamera, verbunden, die das Mikroskopbild der Probenzone aufzeichnet.

Die in den Figuren 1 und 2 dargestellte Ausführungsform hat den Vorteil, dass durch Herausziehen des Mikroskopgehäuserohrs 20 das gesamte Mikroskop als Einheit aus dem Tauchrohr entnommen werden kann, wenn das Tauchrohr in die in Figur 1 unten dargestellte Stellung zurückgezogen ist, ohne dass dabei die sterilen Bedingungen im Reaktorinneren beeinträchtigt werden, da das Reaktorinnere durch das innere Ende des Tauchrohrs und die Dichtungen 9, 9' abgedichtet gehalten wird.

Unterhalb der Probenzone 12 (Figur 2) befindet sich in dem Mikroskopgehäuserohr eine Beleuchtungseinheit, die eine Lichtquelle, ggf. Linsen, und an dem zu der Probenzone 12 weisenden Ende den Objektträgerglaskörper aufweist.

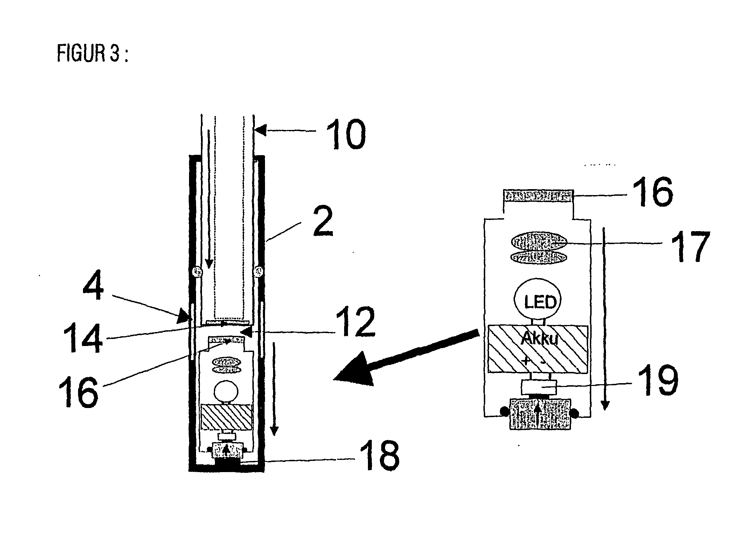

Figur 3 zeigt eine Teilansicht einer weiteren Ausführungsform im Schnitt, wobei hier lediglich das Tauchrohr 2 mit dem Objektivende des darin eingeschobenen Mikroskopaußenrohrs 10 dargestellt ist, wohingegen der Reaktoranschlussport und die Spülkammer zur Vereinfachung der Darstellung fortgelassen sind. In dieser Ausführungsform fehlt ein Mikroskopgehäuserohr. Stattdessen sind die Beleuchtungseinheit und das Mikroskopaußenrohr 10 direkt in dem Tauchrohr 2 aufgenommen. Die Beleuchtungseinheit befindet sich im Reaktorinneren liegenden Ende des Tauchrohrs und wird von einem Magneten 18 am Boden des Tauchrohrs lösbar gehalten. Die Beleuchtungseinheit hat einen beweglich darin gelagerten Kontaktkörper, der auf dem Magneten 18 aufliegt. Der bewegliche Kontakt ist in Anlage an einem Schalter 19 im Inneren der Beleuchtungseinheit. Der Schalter 19 ist mit einer Stromquelle, z.B. einem Akkumulator, verbunden und verbindet die Stromquelle bei Auslösung des Schalters mit der Lichtquelle, vorzugsweise mit einer Leuchtdiode (LED). Oberhalb der Lichtquelle liegt ein Kondensor 17 und darüber ein Objektträgerglaskörper 16, der als Saphirscheibe ausgebildet ist.

Beim Schließen der Probenzone 12 wird das Mikroskopaußenrohr 10 durch einen Antrieb in Richtung auf die Beleuchtungseinheit zu gefahren, bis das Objektivdeckglas 14 mit dafür vorgesehenen Punkten auf dem Objektträgerglaskörper 16 in Berührung kommt. Dadurch wird die Beleuchtungseinheit insgesamt in Richtung auf den Boden des Tauchrohrs gedrückt, wodurch der bewegliche Kontaktkörper auf den Schalter 19 einwirkt und die Lichtquelle bei geschlossener Probenzone 12 aktiviert wird. Da die Lichtquelle nur eingeschaltet wird, wenn die Probenzone 12 geschlossen ist, ist der Stromverbrauch minimal. Die Fixierung der Beleuchtungseinheit am Boden des Tauchrohrs 2 durch eine Befestigungseinrichtung, z.B. einen Magneten, ist wichtig, damit sich die Beleuchtungseinheit beim Öffnen der Probenzone 12 nicht vom Boden ablöst (dies könnte sonst durch adhäsive Kräfte geschehen, die zwischen dem Objektivdeckglas 14 und dem Objektträgerglaskörper 16 und dem von diesen eingeschlossenen Flüssigkeitsfilm wirken). Die Beleuchtungseinheit ist an ihrem der Probenzone zugewandten Ende mit Ausnehmungen versehen, so dass sie dort den Querschnitt des Tauchrohrs 2 nicht vollständig ausfüllt. Aufgrund dieser Ausnehmungen kann die Beleuchtungseinheit mit einem Spezialwerkzeug, beispielsweise einer Zange, gegriffen und aus dem Tauchrohr 2 entfernt werden, wenn das Tauchrohr 2 in der zurückgezogenen Stellung in Reaktoranschlussport und Spülkammer ist und das Mikroskopaußenrohr 10 aus dem Tauchrohr 2 herausgezogen ist.

Durch die Erfindung ist es möglich, während des laufenden Kultivierungsprozesses das Tauchrohr in den Reaktoranschlussport zurückzuziehen und die Mikroskopkomponenten zu entnehmen, wodurch insbesondere die Probenzone durch Austausch von Komponenten, z.B. des Objektträgerglaskörpers, variiert werden kann.

Der Objektträgerglaskörper 16 ist in Figur 3 lediglich schematisch als Saphirglasscheibe dargestellt. Damit zwischen dem Objektivdeckglas 14, dessen Außenfläche eben ist, und der Oberfläche des Objektträgerglaskörpers 16 eine exakt definierte Probenzone gebildet wird, kann der Objektträgerglaskörper 16 an seiner Außenfläche mit Abstandshaltern versehen sein, die bei geschlossener Probenzone in Anlage an das Objektivdeckglas 14 kommen, so dass eine Zwischenschicht mit definierter Dicke gebildet wird.

Um solche Abstandshalter zu realisieren, kann z.B. eine ringförmige Struktur auf dem scheibenförmigen Objektträgerglaskörper aufgebracht sein, wobei dieser Ringkörper aus biokompatiblem Material gebildet sein sollte und nicht komprimierbar sein sollte.

Ein solcher Ringkörper ist in Figur 4 mit dem Bezugszeichen 32 versehen. Ein solcher Ringkörper 32 mit vorgegebener Dicke kann z.B. durch Aufdampfen von Material auf die Saphirglasscheibe 33 gebildet werden. Der Ringkörper 32 kann auch durch Einpolieren einer Vertiefung gebildet werden. Der Ringkörper kann ferner auch aus Edelstahlfolien oder Kunststofffolien ausgeschnitten und auf die Glasscheibe aufgebracht sein. Grundsätzlich ist es auch möglich, den Ring durch Aufdampfen von Saphir auf die Saphirglasscheibe 33 zu bilden. In allen Fällen kann der Ringkörper 32 mit vorgegebener Dicke gebildet werden, so dass eine Probenzone mit vorgegebener Höhe, die durch die Dicke des Ringkörpers 32 als Abstandshalter bestimmt wird, definiert wird. Dadurch kann eine beliebige Höhe der Probenzone vorgegeben werden, und diese Höhe kann relativ einfach (durch Austausch des Ringkörpers) oder durch kompletten Austausch des Objektträgerglaskörpers verändert werden.

Die Herstellungsweise eines äußeren Ringkörpers durch Einpolieren einer Vertiefung ist kostengünstiger als das Aufdampfen, ist aber weniger genau. Ferner können beim Einpolieren der Vertiefung Kratzer entstehen, die die Bildaufnahme stören können. In typischen Fällen wird die Vertiefung mit einer Tiefe von 40 µm gebildet.

Bei den vorgeschriebenen Probenzonen mit einem äußeren Ringkörper als Abstandshalter ist das Volumen der Probenzone bei herangefahrenem Objektivdeckglas gegen das umgebende Kulturmedium abgeschlossen, da das Objektivdeckglas an den Ringkörper anliegt und dieser am Umfang geschlossen ist. Auf diese Weise ist das Medium in der Probenzone ruhiggestellt.

In einer anderen Form der Probenzone kann diese durch eine Mehrzahl von separaten Abstandshaltern 34 oder 35 definiert werden, wie in Figur 4 unten dargestellt. Z.B. können zwei gradlinige Abstandshalter 34 vorgesehen sein, die zwischen sich eine kanalförmige Zone über der Oberfläche des Objektträgerglaskörpers 33 umschließen, wobei die Enden der kanalförmigen Zone offen sind. Auf diese Weise wird, wenn das Objektivdeckglas in Anlage an die geradlinigen Abstandshalter 34 kommt, eine offene Probenzone gebildet. Durch diese offene Probenzone können die Zellen des Kulturmediums strömen. Daher ändert sich die Zusammensetzung des Probenzoneninhalts ständig. Das Gleiche gilt für Ausführungsformen mit mehreren Abstandshaltern 35, die am Umfang eines Rings auf der Saphirglasscheibe 33 angeordnet sind, aber mehrere Unterbrechungen aufweisen, so dass die Probenzone ebenfalls offen gestaltet ist.

Durch die Aufnahme von Bildsequenzen bei offenen Probenzonen ist eine viel höhere Messfrequenz realisierbar, was bei der Messung von schnell wachsenden Organismen von Bedeutung sein kann. Die höhere Messfrequenz ist möglich, da nicht für jedes Bild in der Sequenz die Probenzone geöffnet und geschlossen werden-muss, sondern lediglich nach bestimmten längeren Perioden ein kompletter Austausch des Probenzoneninhalts durch Öffnen und Schließen der Probenzone durchgeführt wird. Diese Ausführungsform der Probenzone unterscheidet sich deutlich von den vorher beschriebenen Probenzonen, die ausschließlich dazu da sind, die Zellen in der Probenzone ruhigzustellen. Bei den offenen Probenzonen wird das Gegenteil erreicht und ermöglicht, einen ständigen Durchfluss durch die Probenzone aufrecht zu erhalten, um Verstopfungen bzw. Ablagerungen zu vermeiden und repräsentative Proben zu gewährleisten. Bei Verwendung lichtstarker Lichtquellen kann die Belichtungszeit der CCD-Kamera soweit verkürzt werden, dass die Zellen, trotz der Bewegung, scharf abgebildet werden.

Bei allen Ausführungsformen der Erfindung kann innerhalb eines Mikroskopaußenrohrs 10 ein Mikroskoptubus, der an seinem vorderen Ende das Objektiv trägt, verschiebbar gelagert sein, so dass der Mikroskoptubus durch Antriebsmittel relativ zu dem Objektivdeckglas 14 an dem Mikroskopaußenrohr 10 verschoben und eingestellt werden kann, um dadurch eine Fokussierung zu ermöglichen. Der Mikroskoptubus ist in Figur 2 mit dem Bezugszeichen 36 versehen.

Mit der oben beschriebenen Beleuchtungseinheit wird die in-situ-Mikroskopvorrichtung zur Durchlicht-Fiellfeldmikroskopie eingesetzt. Alternativ kann auch eine Beleuchtung vorgesehen werden, mit der eine Auflicht-Dunkelfeldmikroskopie ermöglicht wird. Eine derartige Beleuchtung kann z.B. dadurch realisiert werden, dass außerhalb des Tauchrohrs eine externe Lichtquelle vorhanden ist, deren Licht in Lichtleiter eingekoppelt wird, die innerhalb des Mikroskopaußenrohrs nach innen zum Objektivende geführt und durch einen das Objektiv umfassenden Montagering geführt sind. Die lichtabstrahlenden Endflächen der Lichtleiter sind so an dem Montagering ausgerichtet, dass das abgestrahlte Licht schräg auf den Objektträgerglaskörper gerichtet wird, so dass kein direktes oder reflektiertes Licht, sondern nur von den Messobjekten gestreutes Licht in das Objektiv eintritt, wie bei der Dunkelfeldmikroskopie erforderlich. Alternativ können die lichtabstrahlenden Endflächen der Lichtleiter mit ihren Abstrahlwinkeln so eingestellt werden, dass das austretende Licht das Gesichtsfeld gleichmäßig ausleuchtet, wie für die Auflicht-Hellfeldbeleuchtung erforderlich. Ferner kann durch Vorsehen einer monochromatischen Lichtquelle und eines Sperrfilters vor der Kamera auch eine Epi-Fluoreszenzbeleuchtung im Auflicht realisiert werden.

## Patentansprüche

1. In-situ-Mikroskopvorrichtung für Reaktoren mit Reaktoranschlussport (6), umfassend folgende Merkmale:
• ein Tauchrohr (2), das in den Reaktoranschlussport (6) einsetzbar ist und im zum Reaktorinneren weisenden Endbereich eine Probenzone (12) mit einem Einlass (4) aufweist;
• ein koaxial in dem Tauchrohr (2) angeordnetes Mikroskopaußenrohr (10) mit einem äußeren Ende und einem zum Reaktorinneren weisenden inneren Ende und einem am inneren Ende angeordneten Objektivdeckglas (14) sowie einem Objektiv zur Beobachtung der Probenzone (12);
• eine im Tauchrohr (2) gegenüber dem inneren Ende des Mikroskopaußenrohr (10) angeordnete Beleuchtungseinheit mit einer Lichtquelle und einem von der Beleuchtungseinheit getragenen Objektträgerglaskörper (16), wobei die Probenzone (12) durch den Abstand zwischen Objektivdeckglas (14) und Objektträgerglaskörper (16) definiert wird und von der Lichtquelle durch den Objektträgerglaskörper (16) durchleuchtet wird;
• eine außerhalb des Tauchrohrs (2) liegende Kamera (31), die an das äußere Ende des Mikroskopaußenrohrs (10) zur Aufnahme des Bildes der Probenzone (12) gekoppelt ist;
• wobei Objektivdeckglas (14) und Objektträgerglaskörper (16) relativ zueinander beweglich sind, um die Probenzone (12) zu öffnen und zu schließen,
**dadurch gekennzeichnet,**
• **dass** die Relativbewegung von Objektivdeckglas (14) und Objektträgerglaskörper (16) durch eine Bewegung des Mikroskopaußenrohrs (10) im Tauchrohr (2) gegenüber dem Objektträgerglaskörper (16) realisiert wird
• **dass** die Mikroskopvorrichtung eine an den Reaktoranschlussport (6) außen anschließbare Spülkammer (7) mit verschließbaren Öffnungen (8) aufweist, durch die Reinigungsmittel einspeisbar sind
• **dass** das Tauchrohr (2) in axialer Richtung beweglich in dem Anschlussport (6) und der Spülkammer (7) geführt ist und soweit zurückziehbar ist, dass der Einlass (4) des Tauchrohrs (2) in Verbindung mit der Spülkammer (7) steht, um die Probenzone (12) zu reinigen,
• **dass** am Tauchrohr (2) Dichtungsmittel (9, 9') vorgesehen sind, um bei zur Spülkammer (7) zurückgezogenem Tauchrohr (2) den Innenraum des Reaktors gegen die Spülkammer (7) abgedichtet zu halten,
• **dass** ein am äußeren Ende des Mikroskopaußenrohrs (10) einwirkender Antrieb (30) vorgesehen ist
wobei entweder
in dem Tauchrohr (2) ein das Mikroskopaußenrohr (10) und die Beleuchtungseinheit aufnehmendes Mikroskopgehäuserohr (20) angeordnet ist, wobei das Mikroskopgehäuserohr (20) am dem Reaktorinneren zugewandten Ende außer auf einen mit dem Einlass (4) des Tauchrohrs (2) kommunizierenden Einlass für Probenmaterial geschlossen ist und die Probenzone (12) rückwärtig umschließt und wobei die Beleuchtungseinheit im innen liegenden Ende des Mikroskopgehäuserohrs (20) angeordnet ist, und wobei das Mikroskopaußenrohr (10) in axialer Richtung beweglich in dem Mikroskopgehäuserohr (20) gelagert ist und der Antrieb (30) das Mikroskopaußenrohr (10) relativ zum Mikroskopgehäuserohr (20) verschieben kann,
oder
am Boden des Tauchrohrs (2) die Beleuchtungseinheit als separate Einheit lösbar angebracht ist, wobei das Mikroskopaußenrohr (10) unmittelbar in dem Tauchrohr (2) gelagert und in axialer Richtung durch den Antrieb (30) relativ zum Tauchrohr (2) verschiebbar ist,
so dass die Probenzone (12) zwischen Objektivdeckglas (14) und dem Objektträgerglaskörper (16) der Beleuchtungseinheit durch Vorschieben und Zurückziehen des Mikroskopaußenrohrs (10) zu schließen und zu öffnen ist und die Stellung von Objektträgerglaskörper (16) und Objektivdeckglas (14) zueinander durch den Antrieb (30) gesteuert eingestellt werden kann.

2. In-situ- Mikroskopvorrichtung nach Anspruch 1, wobei der Antrieb (30) ein Schrittmotor oder ein geregelter Gleichstrommotor ist.

3. In-situ-Mikroskopvorrichtung nach einem der vorhergehenden Ansprüche, wobei ein Mikroskoptubus (36) verschiebbar in dem Mikroskopaußenrohr (10) gelagert ist und dass ein Antriebsmittel vorgesehen ist, um den Mikroskoptubus (36) in Längsrichtung gesteuert zu verschieben, um das Objektiv an dem Mikroskoptubus in Bezug auf die Probenzone (12) zur Fokussierung einstellbar zu machen.

4. In-situ-Mikroskopvorrichtung nach einem der vorhergehenden Ansprüche, wobei der Objektträgerglaskörper (16) eine Saphirglasscheibe (33) mit ebener Außenfläche aufweist, auf der am Umfang ein ringförmiger Rand (32) mit vorgegebener Dicke gebildet ist, der als Abstandshalter dient, wenn das Objektivdeckglas (14) zum Schließen der Probenzone an den Objektträgerglaskörper (16) bis in Anlage an den ringförmigen Rand (32) herangefahren wird.

5. In-situ-Mikroskopvorrichtung nach Anspruch 4, wobei der ringförmige Rand durch Einpolieren einer Vertiefung in eine Saphirglasscheibe gebildet ist.

6. In-situ-Mikroskopvorrichtung nach Anspruch 4, wobei der ringförmige Rand (32) durch Aufbringen einer ringförmigen Materialschicht gebildet ist.

7. In-situ-Mikroskopvorrichtung nach einem der Ansprüche 1 bis 3, wobei der Objektträgerglaskörper (16) eine Saphirglasscheibe (33) mit ebener Außenfläche aufweist, auf der eine Mehrzahl von Abstandshaltern (34, 35) mit gleicher, vorgegebener Dicke gebildet ist, die als Abstandshalter dienen, wenn das Objektivdeckglas (14) zum Schließen der Probenzone an den Objektträgerglaskörper (16) bis in Anlage an die Abstandshalter herangefahren wird.

8. In-situ-Mikroskopvorrichtung nach Anspruch 7, wobei der Objektträgerglaskörper (16) auf der Außenfläche zwei Abstandshalter (34) aufweist, die zwischen sich eine kanalförmige, zu beiden Enden offene Vertiefung bilden.

9. In-situ-Mikroskopvorrichtung nach einem der vorhergehenden Ansprüche, wobei die Beleuchtungseinheit eine Leuchtdiode als Lichtquelle hat.

10. In-situ-Mikroskopvorrichtung nach Anspruch 1, wobei zum Betrieb des Mikroskops zur Auflicht-Dunkelfeldmikroskopie eine außerhalb des Tauchrohrs angeordnete externe Lichtquelle sowie Lichtleiter vorgesehen sind, die so angeordnet sind, dass sie Licht aus der Lichtquelle aufnehmen und zu einem das Objektiv umgebenden Montagering führen, aus dem ihre lichtabstrahlenden Endflächen herausragen, um das austretende Licht auf die Oberfläche des Objektträgerglaskörpers in der Weise zu richten, dass kein direktes Licht oder reflektiertes Licht, sondern nur von den Messobjekten in der Probenzone gestreutes Licht in das Objektiv eintritt.

11. In-situ-Mikroskopvorrichtung nach Anspruch 1, wobei zum Betrieb des Mikroskops zur Auflicht-Hellfeldmikroskopie eine außerhalb des Tauchrohrs angeordnete externe Lichtquelle sowie Lichtleiter vorgesehen sind, die so angeordnet sind, dass sie Licht aus der Lichtquelle aufnehmen und zu einem das Objektiv umgebenden Montagering führen, aus dem ihre lichtabstrahlenden Endflächen herausragen, wobei der Abstrahlwinkel so gewählt ist, dass das austretende Licht das Gesichtsfeld gleichmäßig, wie für die Auflicht-Hellfeldbeleuchtung erforderlich, ausleuchtet.

12. In-situ-Mikroskopvorrichtung nach Anspruch 11, wobei eine monochromatischen Lichtquelle verwendet wird und dass ein Sperrfilter vor der Kamera angeordnet ist, um eine Epi-Fluoreszenzbeleuchtung im Auflicht zu ermöglichen.

## Claims

1. In-situ microscope device for reactors with reactor connection port, comprising the following features:
- a dip tube (2) which is insertable into the reactor connection port (6) and which comprises, in the end portion pointing to the inside of the reactor, a specimen zone (12) having an inlet (4),
- a microscope external tube (10) arranged coaxially in the dip tube (2), which microscope external tube has an outer end and an inner end facing the inside of the reactor and which comprises at its inner end a lens cover glass (14) as well as a lens for observing the specimen zone (12);
- an illumination unit being arranged in the dip tube (2) opposite to the inner end of the microscope external tube (10), the illumination unit having a light source and a slide glass body (16) carried by the illumination unit, wherein the specimen zone (12) is defined by the space between the lens cover glass (14) and the slide glass body (16) and is illuminated by the light source through the slide glass body (16);
- a camera (31) being located outside of the dip tube (2) and being coupled to the outer end of the microscope external tube (10) for recording the image of the specimen zone (12);
- wherein the lens cover glass (14) and the slide glass body (16) are movable relative to each other in order to open and close the specimen zone (12);
**characterized in that**
- the relative movement of the lens cover glass (14) and the slide glass body (16) is accomplished by movement of the microscope external tube (10) within the dip tube (2) relative to the slide glass body (16);
- the microscope device comprises a rinsing chamber (7), connectable outside to the reactor connection port (6), which rinsing chamber has sealable openings (8) through which cleaning agents can be fed;
- the dip tube is mounted to be movable in axial direction within the reactor connection port (6) and the rinsing chamber (7), and can be pulled back until the inlet (4) communicates with the rinsing chamber (7) in order to clean the specimen zone (12),
- sealing means (9, 9') are provided at the dip tube (2) in order to keep the internal space of the reactor sealed off from the rinsing chamber (7) when the dip tube (2) is pulled back to the rinsing chamber (7);
- a drive (30) is provided which acts on that end of the microscope external tube (10) that lies outside of the reactor;
wherein either
- in the dip tube (2) there is arranged a microscope-housing tube (20) housing the microscope external tube (10) and the illumination unit, wherein the microscope-housing tube (20) is closed at the end facing the inside of the reactor except for an inlet, communicating with the inlet (4) of the dip tube (2), for specimen material, and surrounds the specimen zone (12) from the rear, and wherein the illumination unit is arranged at the inward-lying end of the microscope-housing tube (20), and wherein the microscope external tube (10) is housed movable in axial direction in the microscope-housing tube (20) and the drive (30) is able to shift the microscope external tube (10) relative to the microscope-housing tube (20),
or
- the illumination unit is detachably attached as a separate unit to the base of the dip tube (2), the microscope external tube (10) is housed movable in axial direction directly in the dip tube (2), and is movable in axial direction relative to the dip tube (2) by the drive (30),
such that the specimen zone (12) between the lens cover glass (14) and the slide glass body (16) of the illumination unit can be closed and opened by pushing forward and pulling back the microscope external tube (10), and the relative positioning of the slide glass body (16) and the lens cover glass (14) may be adjusted in a controlled manner by the drive (30).

2. In-situ microscope device according to claim 1, wherein the drive (30) is a step motor or a regulated direct-current motor.

3. In-situ microscope device according to any of the preceding claims, wherein a microscope tube (36) is housed movable in the microscope external tube (10) and wherein a drive means is provided in order to move the microscope tube (36) in longitudinal direction in a controlled manner, in order to be able to set the lens at the microscope tube relative to the specimen zone (12) for focussing.

4. In-situ microscope device according to any of the preceding claims, wherein the slide glass body (16) comprises a sapphire glass plate (33) with a level outer surface on which on the circumference an annular rim (32) of a pre-defined thickness is formed, which serves as a spacer when the lens cover glass (14) is moved towards the slide glass body (16) up against the annular rim (32) until it rests against the spacers, in order to close the specimen zone.

5. In-situ microscope device according to claim 4, wherein the annular rim is formed by polishing in of a recess into a sapphire glass plate.

6. In-situ microscope device according to claim 4, wherein the annular rim (32) is formed by applying an annular material layer.

7. In-situ microscope device according to any of the claims 1 to 3, wherein the slide glass body (16) comprises a sapphire glass plate with a level outer surface on which a plurality of spacers (34, 35) of the same, pre-defined thickness is formed which serve as spacers, when the lens cover glass is moved up against the slide glass body (16) until it rests against the spacers, in order to close the specimen zone.

8. In-situ microscope device according to claim 7, wherein the slide glass body (16) has on the outer surface two spacers (34) which between them form a channel-shaped recess open on both sides.

9. In-situ microscope device according to any of the preceding claims, wherein the illumination unit comprises a light-emitting diode as a light source.

10. In-situ microscope device according to claim 1, wherein, for operating the microscope for direct-light darkfield microscopy, an external light source arranged outside of the dip tube as well as light guides are provided, which light guides are arranged such that they receive light from the light source and guide it to a mounting ring, encompassing the lens, from which their light-radiating end-surfaces project in order to direct the emerging light onto the surface of the slide glass body in such a way that no direct or reflected light, but only light scattered by the measuring objects in the specimen zone enters the lens.

11. In-situ microscope device according to claim 1, wherein, for operating the microscope for direct-light bright-field microscopy, an external light source arranged outside the dip tube as well as light guides are provided, which light guides are arranged such that they receive light from the light source and guide it to a mounting ring, encompassing the lens, from which their light-radiating end-surfaces project, wherein the angle of radiation being chosen such that the emerging light evenly illuminates the field of view, as is necessary for direct-light bright-field illumination.

12. In-situ microscope device according to claim 11, wherein a monochromatic light source is used and a blocking filter is arranged in front of the camera in order to enable an epi-fluorescent illumination in the direct light.

## Revendications

1. Dispositif de microscopie in situ pour des réacteurs avec un orifice (6) de raccordement à un réacteur, comprenant les caractéristiques suivantes :
• un tube plongeur (2), qui peut être inséré dans l'orifice (6) de raccordement au réacteur et qui présente une zone d'échantillonnage (12) avec une entrée (4) dans une zone d'extrémité tournée vers l'intérieur du réacteur ;
• un tube externe de microscope (10), disposé de manière coaxiale dans le tube plongeur (2), avec une extrémité extérieure, une extrémité intérieure tournée vers l'intérieur du réacteur et un verre couvre-objectif (14) disposé à l'extrémité interne ainsi qu'un objectif pour observer la zone d'échantillonnage (12) ;
• une unité d'éclairage disposée dans le tube plongeur (2) en face de l'extrémité interne du tube externe de microscope (10), avec une source de lumière et un corps de verre porte-objet (16) soutenu par l'unité d'éclairage, la zone d'échantillonnage (12) étant définie par la distance entre le verre couvre-objectif (14) et le corps de verre porte-objet (16) et étant éclairée par la source de lumière à travers le corps de verre porte-objet (16);
• un appareil photo (31) situé à l'extérieur du tube plongeur (2), qui est couplé à l'extrémité extérieure du tube externe de microscope (10) pour enregistrer une image de la zone d'échantillonnage (12) ;
• dans lequel le verre couvre-objectif (14) et le corps de verre porte-objet (16) sont mobiles, l'un par rapport à l'autre, pour ouvrir et fermer la zone d'échantillonnage (12),
**caractérisé en ce que**
• le mouvement relatif du verre couvre-objectif (14) et du corps de verre porte-objet (16) est assuré par un mouvement du tube externe de microscope (10) dans le tube plongeur (2) par rapport au corps de verre porte-objet (16),
• le dispositif de microscopie présente une chambre de rinçage qui peut être raccordée extérieurement à l'orifice (6) de raccordement au réacteur et est pourvue d'ouvertures verrouillables (8), à travers lesquelles peuvent être injectés les produits de nettoyage,
• le tube plongeur (2) est guidé de manière mobile dans la direction axiale dans 1"orifice de raccordement (6) et la chambre de rinçage (7) et peut être retiré assez largement pour que l'entrée (4) du tube plongeur (2) soit reliée à la chambre de rinçage (7), afin de nettoyer la zone d'échantillonnage (12),
• le tube plongeur (2) est pourvu de moyens d'étanchéité (9, 9') pour maintenir étanche l'espace interne du réacteur par rapport à la chambre de rinçage (7) lorsque le tube plongeur (2) est retiré vers la chambre de rinçage (7),
• on prévoit un entraînement (30) agissant sur l'extrémité extérieure du tube externe de microscope (10),
dans lequel
on dispose dans le tube plongeur (2) un tube de logement de microscope (20) abritant le tube externe de microscope (10) et l'unité d'éclairage, dans lequel le tube de logement de microscope (20) est fermé à l'extrémité tournée vers l'intérieur du réacteur, sauf sur une entrée du matériau d'échantillon communiquant avec l'entrée (4) du tube plongeur (2), et entoure à l'arrière la zone d'échantillonnage (12), dans lequel l'unité d'éclairage est située à l'extrémité du tube de logement de microscope (20) située à l'intérieur et dans lequel le tube externe de microscope (10) est logé de manière mobile dans la direction axiale dans le tube de logement de microscope (20) et l'entraînement (30) peut déplacer le tube externe de microscope (10) par rapport au tube de logement de microscope (20),
ou dans lequel
on dispose, de manière amovible, l'unité d'éclairage, sous la forme d'une unité séparée, au fond du tube plongeur (2), le tube externe de microscope (10) étant logé directement dans le tube plongeur (2) et pouvant être déplacé par rapport au tube plongeur (2) dans une direction axiale par l'entraînement (30),
de manière à pouvoir fermer et ouvrir la zone d'échantillonnage (12) entre le verre couvre-objectif (14) et le corps de verre porte-objet (16) de l'unité d'éclairage en poussant en avant et en tirant en arrière le tube externe de microscope (10) et à pouvoir régler la position du corps de verre porte-objet (16) et du verre couvre-objectif (14) l'un par rapport à l'autre par commande de l'entraînement (30).

2. Dispositif de microscopie in situ selon la revendication 1, dans lequel l'entraînement (30) est un moteur pas à pas ou un moteur à courant continu asservi.

3. Dispositif de microscopie in situ selon l'une quelconque des revendications précédentes, dans lequel un tube de microscope (36) est monté de manière à pouvoir coulisser dans le tube externe de microscope (10) et dans lequel on prévoit un moyen d'entraînement pour faire coulisser le tube de microscope (36) par commande dans la direction longitudinale, afin de pouvoir régler l'objectif sur le tube de microscope par rapport à la zone d'échantillonnage (12) pour la mise au point.

4. Dispositif de microscopie in situ selon l'une quelconque des revendications précédentes, dans lequel le corps de verre porte-objet (16) présente une plaque de verre en saphir (33) avec une surface externe plane, à la périphérie de laquelle est formé un bord annulaire (32) d'épaisseur prédéterminée, qui sert d'écarteur lorsque, pour fermer la zone d'échantillonnage sur le corps de verre porte-objet (16), le verre couvre-objectif (14) est rapproché jusqu'à venir en appui sur le bord annulaire (32).

5. Dispositif de microscopie in situ selon la revendication 4, dans lequel le bord annulaire est formé par polissage d'une cavité dans une plaque de verre en saphir.

6. Dispositif de microscopie in situ selon la revendication 4, dans lequel le bord annulaire (32) est formé par application d'une couche de matériau annulaire.

7. Dispositif de microscopie in situ selon l'une quelconque des revendications 1 à 3, dans lequel le corps de verre porte-objet (16) présente une plaque de verre en saphir (33) avec une surface externe plane, sur laquelle on forme une pluralité d'écarteurs (34, 35) d'épaisseur prédéterminée identique, qui servent d'écarteurs lorsque, pour fermer la zone d'échantillonnage sur le corps de verre porte-objet (16), le verre couvre-objectif (14) est rapproché jusqu'à venir en appui sur les écarteurs.

8. Dispositif de microscopie in situ selon la revendication 7, dans lequel le corps de verre porte-objet (16) présente deux écarteurs (34) sur la surface externe, lesquels écarteurs formant entre eux une cavité en forme de canal ouverte aux deux extrémités.

9. Dispositif de microscopie in situ selon l'une quelconque des revendications précédentes, dans lequel l'unité d'éclairage a une diode électroluminescente comme source de lumière.

10. Dispositif de microscopie in situ selon la revendication 1, dans lequel on prévoit, pour le fonctionnement du microscope en microscopie à fond noir par réflexion, une source de lumière externe disposée à l'extérieur du tube plongeur ainsi que des fibres optiques, qui sont aménagées de manière à absorber de la lumière issue de la source de lumière et à l'acheminer à un anneau de montage entourant l'objectif, dont les surfaces terminales photoémettrices dépassent pour diriger la lumière sortante sur la surface du corps de verre porte-objet de sorte qu'il n'y ait pas de lumière directe ou de lumière réfléchie qui pénètre dans l'objectif, si ce n'est seulement de la lumière dispersée par les objets mesurés dans la zone d'échantillonnage.

11. Dispositif de microscopie in situ selon la revendication 1, dans lequel on prévoit, pour le fonctionnement du microscope en microscopie à fond clair par réflexion, une source de lumière externe disposée à l'extérieur du tube plongeur ainsi que des fibres optiques, qui sont aménagées de manière à absorber la lumière issue de la source de lumière et à l'acheminer à un anneau de montage entourant l'objectif, dont les surfaces terminales photoémettrices dépassent, l'angle de rayonnement étant choisi de sorte que la lumière sortante éclaire uniformément le champ visuel comme l'exige l'éclairage à fond clair par réflexion.

12. Dispositif de microscopie in situ selon la revendication 11, dans lequel on utilise une source de lumière monochromatique et l'on dispose un filtre d'arrêt devant l'appareil photo pour permettre un éclairage à épifluorescence en lumière réfléchie.
